# EUROPEAN PATENT APPLICATION

(11) **EP 1 350 851 A1**
(43) Date of publication of application: **08.10.2003**
(21) Application number: 01271113.1
(22) Date of filing: 12.12.2001
(51) Int. Cl.: C12Q 1/68, C12N 15/09, G01N 33/53, G01N 27/62, G01N 33/566, C12M 1/00

(54) **METHOD OF DETECTING POLYMORPHISM IN DNA BY USING MASS SPECTROSCOPY**

(30) Priority: 12.12.2000 JP 2000378091
(71) Applicant: CHUGAI SEIYAKU KABUSHIKI KAISHA, Tokyo, 115-8543 (JP)
(72) Inventor: INOKO, Hidetoshi, Atsugi-shi, Kanagawa 243-0034 (JP); TAMIYA, Gen, Isehara-shi, Kanagawa 259-1143 (JP); NAKAJIMA, Kenji, Isehara-shi, Kanagawa 259-1138 (JP); KIMURA, Naoki, Chiba-shi, Chiba 266-0015 (JP); NAGASHIMA, Renpei, c/o CHUGAI SEIYAKU KABUSHIKI K., Tokyo 104-8301 (JP); MORIKAWA, Minoru, c/o CHUGAI SEIYAKU KABUSHIKI K., Tokyo 104-8301 (JP); OKAMOTO, Kouichi, Isehara-shi, Kanagawa 259-1132 (JP)
(74) Representative: Harding, Charles Thomas
(86) International application number: JP0110892
(87) International publication number: WO02050307

(57) **Abstract**

In the detection of a genetic polymorphism using a mass spectrometer, a genomic DNA containing target polymorphism can be separated from a DNA sample and bound at the same time onto a platform by the following steps: binding an oligonucleotide that hybridizes with the genomic DNA containing the target polymorphism to the platform; and then applying the genomic DNA sample to the platform . Thus, a target polymorphism can be more efficiently detected. According to the method of the present invention, polymorphisms in a large number of specimens can be quickly and comprehensively detected.

SEQUENCE LISTING

<110> CHUGAI SEIYAKU KABUSHIKI KAISHA

<120> METHOD OF DETECTING POLYMORPHISMS IN DNA USING MASS SPECTROMETRY

<130> C1-A0017P

<140>
<141>

<150> JP 2000-378091
<151> 2000-12-12

<160> 7

<170> PatentIn Ver. 2.1

<210> 1
<211> 25
<212> DNA
<213> Artificial Sequence

<220>
<223> Description of Artificial Sequence:an artificially
      synthesized DNA sequence

<400> 1
cctagagatt cctccgtatt agttc                                        25


<210> 2
<211> 25
<212> DNA
<213> Artificial Sequence

<220>
<223> Description of Artificial Sequence:an artificially
      synthesized DNA sequence

<400> 2
tccctgtgga tgtcaagaat ctttt                                          25


<210> 3
<211> 20
<212> DNA
<213> Artificial Sequence

<220>
<223> Description of Artificial Sequence:an artificially
      synthesized DNA sequence

<400> 3
aatacggagg aatctctagg                                                20


<210> 4
<211> 40
<212> DNA
<213> Artificial Sequence

<220>
<223> Description of Artificial Sequence:an artificially
      synthesized DNA sequence

<400> 4
tttttttttt aaaaaaaaaa aaaaaaaaaa aaaaaaaaaa                          40


<210> 5
<211> 35

<212> DNA
<213> Artificial Sequence

<220>
<223> Description of Artificial Sequence:an artificially
      synthesized DNA sequence

<400> 5
tttttttttt cctagagatt cctccgtatt agttc                                35


<210> 6
<211> 50
<212> DNA
<213> Artificial Sequence

<220>
<223> Description of Artificial Sequence:an artificially
      synthesized DNA sequence

<400> 6
tttttttttt tttttttttt tttttttttt tttttttttt tttttttttt                50


<210> 7
<211> 100
<212> DNA
<213> Artificial Sequence

<220>
<223> Description of Artificial Sequence:an artificially
      synthesized DNA sequence

<400> 7
tttttttttt tttttttttt tttttttttt tttttttttt tttttttttt tttttttttt    60
tttttttttt tttttttttt tttttttttt tttttttttt                          100

## Description

### Technical Field

The present invention relates to a system for efficiently identifying polymorphisms in genome using mass spectrometry.

### Background Art

Due to the progress in genome analysis techniques, the complete human genome sequence is now being deciphered. Furthermore, using the identified genome sequence information, differences in the genome sequence among individuals, i.e., genetic polymorphism is being actively determined. The determination of genetic polymorphism enables determination of disease related genes and disease causing genes, as well as target genes for drug development. Therefore, in recent years, determination of genetic polymorphism has become one of the most important issues in the field of medical treatment and diagnosis.

Conventionally, PCR-RFLP (Restriction Fragment Length Polymorphism) method is used for determining genetic polymorphism. The principle of this method involves the procedure of: (1) reacting a nucleotide sequence-specific restriction enzyme to PCR amplified product; (2) subjecting the product to electrophoresis; and (3) detecting the presence or absence of cleavage due to differences in the nucleotide sequences as a difference in molecular weight. However, this method has a few drawbacks: a restriction enzyme specifically recognizing a polymorphic site does not always exist; and this method is not suited for a large number of samples since restriction enzymes to be used will differ depending on the targeted SNPs.

PCR-SSPC (Single-Strand Conformation Polymorphism) method has also been used for the detection of genetic polymorphism. When a DNA denatured to single strands is returned to nondenaturing conditions, it forms higher-order molecular structures, such as a hairpin loops. This intramolecular structure is greatly affected and altered by even a single nucleotide mutation. The PCR-SSPC method is based on the principle of detecting structural differences such as the differences in mobility in polyacrylamide gel electrophoresis under nondenaturing conditions. However, there are certain problems with this method. This method fails to detect all polymorphisms due to difficulty in detecting all such mutations and difficulty in obtaining reproducible data due to changes in the mobility because of electrophoretic conditions.

Furthermore, genetic polymorphism has also been analyzed using automatic fluorescence sequencer. According to this method, first, primers are designed to sandwich a polymorphic microsatellite repeat sequence between the primers, and the primers are modified with fluorescence. Next, PCR is performed using these primers, the obtained product is electrophoresed on automatic fluorescence sequencer, and by measuring their chain lengths using a standard DNA as an indicator, polymorphism in the repeat sequence is detected. Recently, with the development of sequencers capable of processing a large number of samples, this method has become widely used, and is an effective means for detecting microsatellite polymorphisms. However, this method has certain problems. For example, the exact chain length of a sample cannot be specified and polymorphism of microsatellites with a repeat unit of 1 or 2 bases is difficult to determine via this method.

On the other hand, detection of polymorphism using mass spectrometry is also being performed. Until recently, mass spectrometry had been used mainly for testing the purity of synthetic oligonucleotides and not for detecting polymorphism due to reasons such as inability to quickly analyze a large number of samples and the assumed limit of the molecular weight of DNA that can be detected with this method. Testing the purity using mass spectrometry, a synthetic oligonucleotide specimen is directly applied with a matrix solution to a stainless steel platform or to a platform maintaining equivalent conductivity, dried, and then peak detection is carried out by matrix-assisted laser desorption ionization time-of-flight mass spectrometry (MALDI-TOF MS).

It is only recent that detection of single nucleotide polymorphism (SNP) using a mass spectrometer is being performed. According to this method, a genomic fragment to be detected is immobilized onto a platform by a silicone dioxide derivatization reaction, which is then subjected to the polymerase chain reaction using Primer-Oligo Based Extension Primers, followed by detection of the reactants using a mass spectrometer, and thereby determining the single nucleotide polymorphism (Kai Tang et al., Proc. Natl. Acad. Sci. USA Vol. 96, pp. 10016-10020, (1999)).

However, when measuring a large number of specimens, these conventional methods for detecting genetic polymorphisms using a mass spectrometer require a lot of time and effort. This is because a DNA sample containing polymorphism to be detected has to be prepared and then directly applied to a platform to measure the molecular weight.

### Disclosure of the Invention

This need in the artled to the present invention, the objective of which is to provide a method that enables efficient detection of genetic polymorphism using a mass spectrometer. Another objective of this invention is to provide a method for quickly and comprehensively detecting genetic polymorphisms in a large quantity of specimens.

The present inventors carried out extensive research to achieve the above-mentioned objectives. As a result, the present inventors discovered that genomic DNA containing target polymorphism can be separated from a DNA sample on a platform and also captured on the platform in the detection of genetic polymorphism using a mass spectrometer by binding oligonucleotides that hybridize with genomic DNA containing the target polymorphism on the platform and by applying the genomic DNA sample to this platform; which method finally enables efficient detection of the target polymorphism.

Considerable time and effort was required to detect polymorphism in previous methods because genomic DNA containing target polymorphism was separated in advance and then directly applied to a platform to detect the polymorphism. On the other hand, according to the method of the present invention, oligonucleotides that hybridize to genomic DNA containing target polymorphism are bound to a platform. Therefore, even when DNAs other than the genomic DNA containing the target polymorphism are included in a DNA sample, the target DNA can be separated and at the same time captured on the platform by a hybridization reaction. Thus, according to the method of the present invention, there is no need to separate in advance the genomic DNA containing the polymorphism before applying the DNA to the platform. As a result, efficient detection of the polymorphism can be performed. This is especially effective for detecting polymorphisms of many samples, such as, comprehensively determining the variation of genetic polymorphism of a sample group of 100 to 1000 people, identifying a genetic marker (e.g., polymorphic microsatellite) necessary for a disease correlation analysis of a certain disease group, etc.

This invention was completed based on the above-mentioned findings and provides a method for separating and capturing DNA that contains target polymorphism utilizing hybridization reaction on a platform in the detection of polymorphisms in DNA using mass spectrometry.

More specifically, the present invention provides:
(1) a method for detecting polymorphism in a DNA, which comprises the steps of:
   (a) preparing from a subject, a DNA sample containing a polymorphic site and neighboring region thereof;
   (b) hybridizing the above-mentioned DNA sample to an oligonucleotide fragment that can specifically hybridize to a DNA containing a target polymorphism, wherein said oligonucleotide fragment is immobilized on a platform; and
   (c) detecting by mass spectrometry the DNA hybridized to the oligonucleotide fragment immobilized on the platform;
(2) the method of (1), wherein the oligonucleotide fragment immobilized on the platform has a nucleotide sequence capable of specifically hybridizing to the nucleotide sequence of the neighboring region of the polymorphism to be detected;
(3) the method of (1), wherein the DNA sample of step (a) can be obtained by: (i) performing polymerase chain reaction with a genomic DNA derived from a subject as a template using a primer pair containing a specifically cleavable site in at least one of the primers, which primer pair is designed to sandwich the polymorphic site between the primers, (ii) cleaving the obtained amplified product at the specifically cleavable site, and (iii) adding an arbitrary DNA to the amplified product after the cleavage, and wherein the oligonucleotide of step (b) is an oligonucleotide specifically hybridizing to the arbitrary DNA;
(4) the method of (3), wherein the specifically cleavable site is a restriction enzyme site;
(5) the method of any one of (1) to (4), wherein the polymorphism is a microsatellite;
(6) the method of any one of (1) to (5), wherein the DNA sample containing the polymorphic site and neighboring region thereof is prepared using the polymorphic site of a plurality of different loci of the same person as the target, and the DNA sample is simultaneously hybridized to the oligonucleotide immobilized on the same platform;
(7) the method of any one of (1) to (5), wherein the DNA sample containing the polymorphic site and neighboring region thereof is prepared using the polymorphic site of the same loci of a plurality of people as the target, and the DNA sample is simultaneously hybridized to the oligonucleotide immobilized on the same platform;
(8) a platform to be used in the method of (3), which is immobilized with an oligonucleotide that specifically hybridizes to the arbitrary DNA;
(9) a platform to be used in the method of (6), which is immobilized with an oligonucleotide fragment that hybridizes to the DNA sample containing the polymorphic site and neighboring region thereof, and which sample has been prepared using as the target the polymorphic site of a plurality of different loci of the same person;
(10) a platform to be used in the method of (7), which is immobilized with an oligonucleotide fragment that hybridizes to the DNA sample containing the polymorphic site and neighboring region thereof, and which sample has been prepared using as the target the polymorphic site of the same loci of a plurality of people;
(11) the platform of (9) or (10), wherein the oligonucleotide fragment has a sequence that specifically hybridizes to the sequence of the neighboring region of the polymorphism;
(12) the platform of any one of (8) to (11), wherein the polymorphism is a microsatellite; and
(13) the platform of any one of (8) to (12), which is a glass sheet.

The present invention provides a method for efficient detection of polymorphism in DNA using a mass spectrometer. In the present invention, first, a DNA sample containing a polymorphic site and neighboring region thereof is prepared from a subject (step (a)).

There is no particular limitation on the polymorphism that can be detected according to the method of the present invention. Examples of polymorphisms include single nucleotide polymorphisms (SNPs), microsatellites, etc; however, for correlation analysis wherein vast genomic regions are used as the target, microsatellites are especially preferable. When performing a correlation analysis that covers the entire genomic region of human utilizing microsatellites, the objective may be accomplished with the use of microsatellites from approximately 30,000 sites. However, when single nucleotide polymorphisms are used, approximately 100,000 sites are considered necessary. Therefore, when performing a correlation analysis targeting vast genomic regions employing the method of this invention, target microsatellites are preferably used because the number of polymorphisms required as the detection target can be decreased.

A DNA sample containing a polymorphic site and neighboring region thereof can be prepared from a subject, as follows. Specifically, first, a microsatellite repeat sequence is detected via computer from the human genome sequence and primers are set so that they sandwich the repeated sequence and the chain length of a unit of the replication sequence (amplicon) is 200 to 300 bp. Next, a DNA sample of a desired size is obtained by performing PCR with the genomic DNA of the subject as a template and using the primers set in this manner.

In this invention, a "neighboring region" of a polymorphic site indicates a DNA region adjacent to the 5'-side and 3'-side of the polymorphic site on the genomic DNA. The size of a DNA fragment that can be analyzed using a mass spectrometer is generally 200bp to 300 bp. Therefore, according to this invention, a "region neighboring a polymorphic site" which is the target for performing gene amplification by setting primers is usually 300 bp or less, preferably 200 bp or less from the polymorphic site.

Next, according to the method of this invention, an oligonucleotide fragment immobilized on a platform which fragment can specifically hybridize to a DNA containing a target polymorphism is provided; and the above-mentioned DNA sample is hybridized to the fragment (step (b)).

In this step, a DNA containing a target polymorphism is captured on a platform simultaneously with the separation of the DNA from the DNA sample. Therefore, compared to conventional methods wherein DNA containing a target polymorphism had been prepared in advance and then applied, in this method time and effort is markedly reduced. This is especially remarkable when detecting polymorphisms in a large quantity of specimens. According to the present invention, even when applying a DNA sample consisting of a mixture of DNAs containing a plurality of polymorphisms to a platform on which a plurality of oligonucleotides targeting each of the plurality of polymorphisms are bound as different dots, DNA corresponding to each of the oligonucleotides are separated on the platform. Therefore, depending on the position of hybridization, one can differentiate a DNA to the polymorphism it corresponds. A quick and comprehensive detection of polymorphisms in a large quantity of specimens is enabled by the present method.

There are no particular limitations on the "platform" to be used in the method of the present invention as long as it is a material on which oligonucleotides can be immobilized; however, it is preferably a material in the form of a sheet. Since DNAs used as the detection target is ionized, materials having electroconductivity are used as the platform to bind oligonucleotides. There are no particular limitations on the platform as long as it has electroconductivity. For example, glass sheet immobilized onto an electroconductive sheet can be preferably used as a platform in this invention. Examples of a preferable conductive sheet include a stainless steel sheet. The glass sheet is preferably coated with polycarbodiimide.

There are no particular limitations on the oligonucleotide to be immobilized onto a platform as long as it is an oligonucleotide fragment that can specifically hybridize to a DNA containing a target polymorphism. Herein, the phrase "specifically hybridize" means that the hybridization substantially occurs with DNAs that contain the target polymorphism in the DNA sample, and that no hybridization substantially occurs with other DNAs. The oligonucleotide preferably has a nucleotide sequence capable of specifically hybridizing to the nucleotide sequence of the neighboring region of the polymorphism to be detected. As long as specific hybridization is possible, the oligonucleotides do not necessary have to be completely complementary to the nucleotide sequence of the neighboring region of the polymorphism to be detected.

The oligonucleotide to be immobilized on a platform is not limited to any one kind, and may be a mixture of multiple kinds of oligonucleotides that are complementary to the genomic fragment within the region to be identified. In a preferred embodiment of the present invention, the oligonucleotide to be immobilized on a platform can be designed to yield genetic information from the same person. For example, oligonucleotides targeting many polymorphic sites (e.g., 10, 000 to 30,000 sites) maybe immobilized on one or multiple platforms as a single set to identify polymorphisms in a microsatellite of the same person. Simultaneous application and hybridization of a DNA sample prepared from a subject to such multiple kinds of oligonucleotides on the same platform enables quick and comprehensive detection of a plurality of polymorphisms in the same person. Consequently, diseases that the subject may develop or has developed may be readily identified by binding oligonucleotides targeting a plurality of polymorphisms relating to a plurality of diseases on a platform. Thus, such platforms are especially useful for genetic diagnosis.

Furthermore, in another preferred embodiment of the present invention, genetic information relating to a specific polymorphism in a sample group of a plurality of people can be obtained quickly and comprehensively by binding the same oligonucleotide as multiple dots on a platform. Such an embodiment is especially effective for performing disease correlation analysis using genetic polymorphisms (for example, microsatellites and SNPs).

When an arbitrary DNA is added to the terminus of a DNA containing a polymorphism and neighboring region thereof during the preparation of a DNA sample from a subject, the polymorphism of a target DNA can be detected using a platform immobilized with oligonucleotides that specifically hybridize to the arbitrary DNA. In this case, the DNA sample can be obtained by: (i) performing PCR with genomic DNA derived from the subject as the template using a primer pair which contains a specific cleavable site in at least one of the primers and which primer pair is designed to sandwich the polymorphic site between the primers; (ii) cleaving the obtained amplified product at the specific cleavable site; and (iii) adding an arbitrary DNA to the amplified product after the cleavage. The specific cleavable site in the DNA sample is preferably a restriction enzyme site. Preferable restriction enzyme sites are those that are present only in low frequency in the genome, such as, the NotI site. There are no particular limitations on the arbitrary DNA added to the amplification product after the cleavage treatment, and for example, polyA sequence may be preferably used.

The length of an oligonucleotide that is bound to a platform is generally 5 to 200 bases, preferably 10 to 130 bases, and more preferably 15 to 100 bases. Immobilization of the oligonucleotides to the substrate platform can be performed chemically or non-chemically. An example of a chemical immobilization method is the carbodiimide method (Unexamined Published Japanese Patent Application No. (JP-A) 2000-146978), while an example for a non-chemical immobilization method is the polylysine method (P. O. Brown's Lab.: http://cmgm.stanford.edu/pbrown/).

The hybridization reaction between an oligonucleotide on the platform and a DNA sample can be performed under conditions described in the Examples. However, it is well known to those skilled in the art that the hybridization conditions may vary depending on factors, such as the length of the oligonucleotide.

Next, in the method of this invention, the DNA hybridized to the oligonucleotide fragment immobilized on the platform is detected by mass spectrometry (step (c)).

There are no limitations on the method of mass spectrometry as long as the absolute molecular weight can be determined; for example, MALDI-TOF MS is preferred. Samples for MALDI (Matrix Assisted Laser Desorption/Ionization) are prepared as homogeneous mixtures with large amounts of matrix. The matrix absorbs nitrogen laser beam, ultraviolet light (wavelength=337 nm) and converts light to thermal energy. At this time, a small portion of the matrix is rapidly heated and is vaporized with the sample. Time of Flight Mass Spectrometry (TOF MS) is a method for mass spectrometry utilizing the difference in the flight time of ions due to difference in the mass-to-charge ratio m/z, wherein z denotes ionic charge and m denotes ionic mass. In contrast to other mass spectrometric methods , such as GPC and LALLS, wherein relative molecular weights are determined, MALDI-TOF is characterized by its ability to yield absolute molecular weights.

A DNA sample to be detected by MALDI-TOF MS does not necessarily require high-temperature treatment (such as at 90°C), or rapid cooling treatment of the non-chemically bonded double strand as pretreatments. However, these pretreatments are preferable for elevating specimen sensitivity. When performing mass spectrometry, an appropriate matrix solution is added to the platform hybridized with DNA in order to dry and immobilize the DNA on the platform.

In mass spectrometry, the flight time of an ionized specimen (DNA hybridized to an oligonucleotide on the platform) differs depending on its molecular weight, and different flight times are detected as different peak positions. The higher the molecular weight of the specimen, the shorter the flight time will be, and conversely, the smaller the molecular weight, the longer the flight time. Therefore, the molecular weight of a specimen can be determined from the position of the detected peak, and as a result, the polymorphism in the specimen (number of microsatellite repeats and the type of nucleotide in a single nucleotide polymorphism) can be identified.

### Brief Description of the Drawings

Fig. 1 shows the result of a MALDI-TOF MS measurement of 200 bp dsDNA (PCR product) using a glass sheet.
Fig. 2 shows the hybridization signal when the oligonucleotide of SEQ ID NO: 1 was immobilized using a stainless steel sheet as the platform.
Fig. 3 shows the hybridization signal when the oligonucleotide of SEQ ID NO: 2 was immobilized using a stainless steel sheet as the platform.
Fig. 4 shows the immobilization of a cover glass to a sample slide made from a stainless steel sheet.
Fig. 5 shows the hybridization signal when the oligonucleotide of SEQ ID NO: 4 or 5 (negative control) was immobilized using a glass sheet as the platform.

### Best Mode for Carrying out the Invention

The present invention is specifically illustrated below with reference to Examples, but it is not to be construed as being limited thereto.

### [Example 1] MALDI-TOF MS measurement of 200 bp dsDNA (PCR product) using glass sheet

### (1) PCR reaction

The reaction mixture solution (x 1) used for the PCR was as follows (the amounts for x100 are shown in parentheses).

| | |
|---|---|
| Template DNA 10 ng (pBluescript II SK(+) plasmid DNA) | 1.0 µL (100 µL) |
| dH₂O | 13.4 µL (1340.0 µL) |
| 10x PCR Reaction Buffer (Applied Biosystems) | 2.0 µL (200.0 µL) |
| dNTP (2.0 mM each) (Applied Biosystems) | 2.5 µL (250.0 µL) |
| Forward and Reverse Primers Mix (20 µM each) | 1.0 µL (100.0 µL) |
| AmpliTaqGold (Applied Biosystems) | 0.1 µL (10.0 µL) |
| Total Volume | 20.0 µL (2000.0 µL) |

Using a Thermalcycler Cycle PE9700 (Applied Biosystems), PCR was performed under a condition of "95°C for 9 min"; 40 cycles of "96°C for 45 sec, 60°C for 45 sec, 72°C for 1 min"; and then "72°C for 5 min, and termination at 4°C".

### (2) Purification by MinElute PCR Purification kit

The obtained PCR product was purified according to the instruction of QIAGEN MinElute PCR Purification kit. Three-hundred µL of the PCR product per column was passed through the columnand eluted with 10 µL of dH₂O.

### (3) Ethanol precipitation

Ethanol precipitation was carried out by adding 46.9 µL of 10 M ammonium acetate, 750.0 µL of 100% ethanol, and 0.5 µL of glycogen to 300 µL of the above-mentioned QIAGEN purified product (6 sets (60 µL) + dH₂O 240 µL). The mixture was left standing at -20°C for 20 min, centrifuged at 12,000 rpm for 15 min, the obtained precipitate was rinsed with 70% ethanol followed by further centrifugation at 12,000 rpm for 15 min, dried for 10 min, and finally eluted with 15 µL of dH₂O.

### (4) Quantitative determination using PicoGreen dsDNA Quantitation kit

Quantitative determination was carried out according to the instruction of PicoGreen dsDNA Quantitation kit (Molecular Probes) .

### (5) Mixing with matrix

A glass slide (Code. #000: 0.04 mm thick) was immobilized to a stainless steel sheet (sample slide) with a double-sided adhesive tape.

Furthermore, a matrix was prepared by dissolving 0.0974 g of 0.7 M 3-hydroxypyridine-2-carboxylic acid (3-hydroxy-2-picolinic acid) (MW 139.11) and 0.010583 g of 0.07 M diammonium hydrogen citrate (MW 226.2) in 1 ml of 50% acetonitrile.

The matrix and sample were mixed in a 1:1 ratio, and 0.5 µL thereof was spotted onto the glass. The sample slide was spontaneously dried (crystallized) , introduced into a mass spectrometer for measurement.

### (6) MALDI-TOF mass spectrometry

Measurement was conducted using KRATOS KOMPACT MALDI 4 (Shimadzu) . The measurement conditions for KOMPACT MALDI 4 are shown below.

| | |
|---|---|
| Flight path (ion flight path) | Linear |
| Polarity (ion polarity) | Negative |
| Mass (ion acceleration voltage) | 20 kV |
| Profiles (number of measurements per ion sample) | 50 |
| Aim (full range sample irradiation: 1-1000) | 1-1000 |
| Power (laser power) | 140-170 (when measuring 200 bp) |
| Accumulate | 10 |
| Average | 1 |

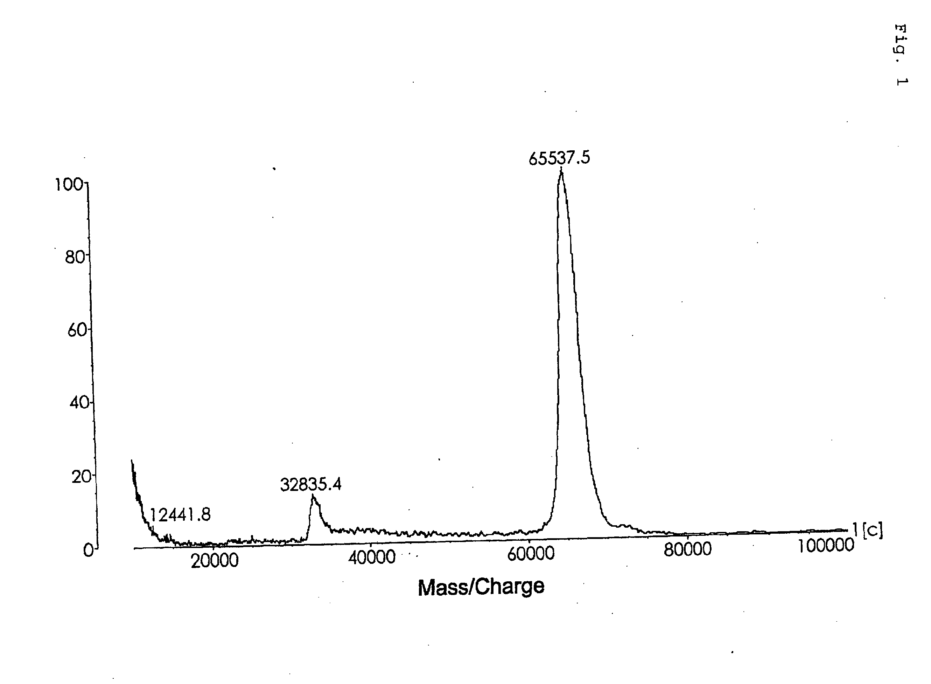

Because of the measurement, a peak showing remarkably high resolution was obtained for a DNA molecule having a high molecular weight, i.e., 200 bp (Fig. 1). This shows that a glass sheet, which is an insulator and had been considered inappropriate for the use in mass spectrometry, is adequately applicable for mass spectrometry. At the same time, the result suggests that a DNA sample hybridized on a glass sheet can be directly subjected to mass spectrometry in that state.

### [Example 2] Mass spectrometry of DNA molecule hybridized on a stainless steel sheet

### (1) Immobilization of nucleic acids

A stainless steel sheet (stainless steel sheet; manufactured by KRATOS ANALYTICAL Corp.) was carbodiimidated according to the method of JP-A 2000-146978.

Oligonucleotides having the nucleotide sequences shown in SEQ ID NO: 1 (calculated value; MW 7583) and SEQ ID NO: 2 (calculated value; MW 7638), respectively, were dissolved in a buffer to a concentration of 100 pmol/µL to produce DNA solutions. Using a Pipetteman, 1 µL each of the above DNA solutions was spotted to a designated position on the carbodiimidated stainless steel sheet.

Next, the sheet was dried at 37°C for 30 min, soaked in buffer A (0.2 M sodium chloride, 0.1 M Tris-HCl (pH 7.5) , 0.05% Triton X-100) containing 3% BSA (bovine serum albumin), and dried at 37°C for 15 min. Then, this carbodiimidated stainless steel sheet was washed with TE buffer (10 mM Tris-HCl (pH 7.2)/1 mM EDTA), and dried at 37°C for 15 min.

### (2) Hybridization

Twenty-five µL of hybridization solution [5x SSC (SSC: 1.5 M NaCl, 0.15 M sodium citrate), 10% dextran, the probe of SEQ ID NO: 3 (calculated value; MW 6190)] was placed on the above-mentioned carbodiimidated stainless steel sheet at the part immobilized with the DNA, and the sheet was heated overnight using a 40°C water bath.

### (3) Post-hybridization

After hybridization, the hybridization solution was lightly absorbed from the carbodiimidated stainless steel sheet, post-hybridization washing was carried out, and non-specifically adsorbed probes were removed.

Conditions for the post-hybridization washing were: (i) 2x SSC, 0.1% SDS, at room temperature for 5 min, twice; (ii) 0.2x SSC, 0.1% SDS, at 40°C for 5 min, twice; (iii) 2x SSC, at room temperature for 5 min; and (iv) 0.3 M aqueous ammonium citrate solution, at room temperature for 15 sec.

### (4) Detection of hybridization

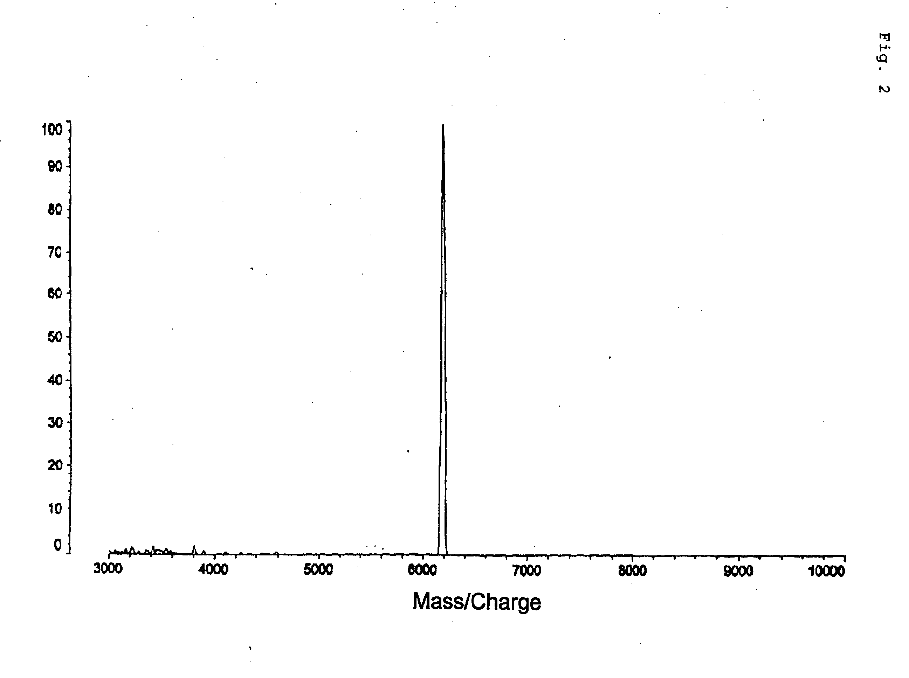

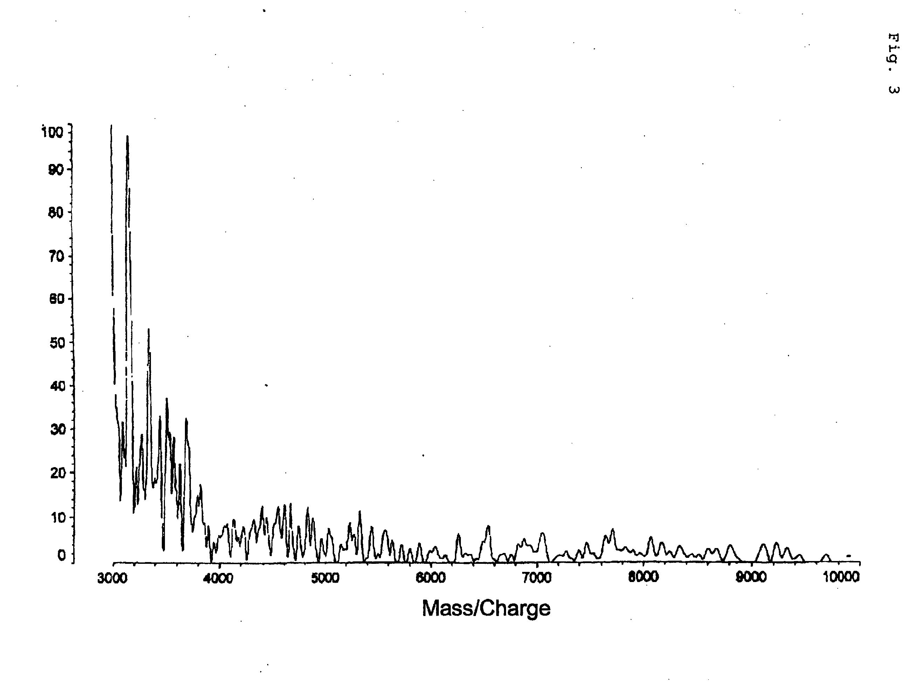

The carbodiimidated stainless steel sheet thus obtained was measured using KOMPACT MALDI 2 (Shimadzu Corp.). The results are shown in Fig. 2 and Fig. 3.

According to the results presented in Fig. 2 and Fig. 3, the method for detecting nucleic acids of the present invention was revealed to allow specific detection of the probe nucleic acid (observed value; at the position of MW 6185) as an extremely clear signal. On the other hand, no signal was detected from the spot of SEQ ID NO: 2.

### [Example 3] Mass spectrometry of DNA molecules hybridized on cover glass

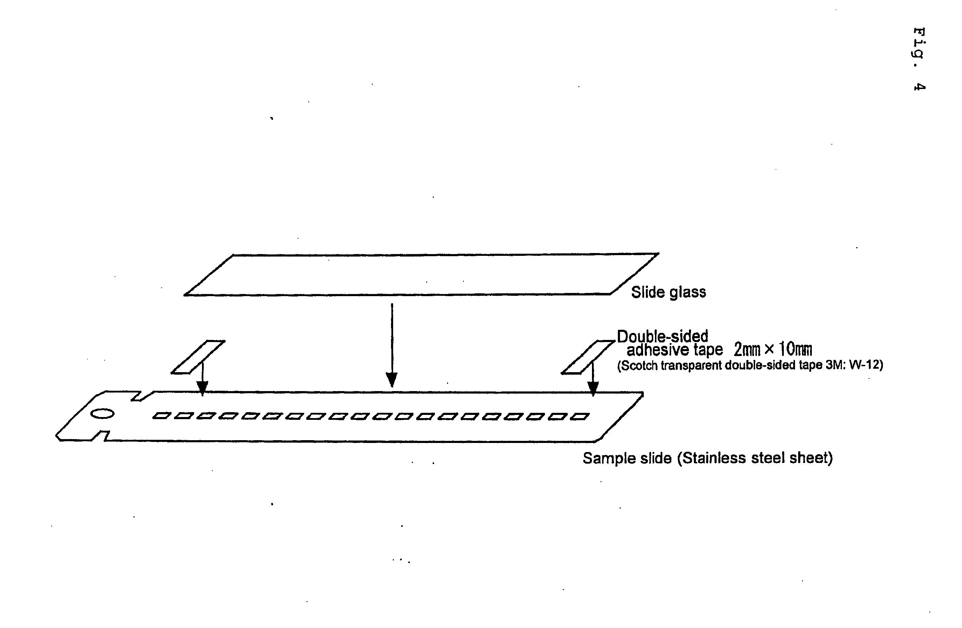

### (1) Immobilization of nucleic acids (Fig. 4)

Cover glass #000 (Matsunami Glass Corp.) was carbodiimidated according to the method of JP-A 2000-146978.

Oligonucleotides having the nucleotide sequences indicated in SEQ ID NO: 4 (Capture oligomer) and SEQ ID NO: 5 (negative control), respectively, were dissolved in buffer to a concentration of 120 pmol/µL to yield DNA solutions. Using a Pipetteman, 0.5 µL each of the aforementioned DNA solutions and buffer were spotted onto the designated position on the carbodiimidated cover glass.

Next, immobilization procedure was carried out, the glass was soaked in buffer A (0.2 M sodium chloride, 0.1 M Tris-HCl (pH 7.5), 0.05% Triton X-100) containing 3% BSA (bovine serum albumin), and dried at 37°C for 15 min. Then, this carbodiimidated cover glass was washed with TE buffer (10 mM Tris-HCl (pH 7.2)/1 mM EDTA), and dried at 37°C for 15 min. The buffer without the addition of oligonucleotide (DNA(-)) was used as the control.

### (2) Hybridization

Ten µL of hybridization solution [5x SSC (SSC: 1.5 M NaCl, 0.15 M sodium citrate), 10% dextran, the probe of SEQ ID NO: 6 or SEQ ID NO: 7] was placed on the above-mentioned carbodiimidated cover glass at the part immobilized with the DNA, and the glass was heated overnight in a 30°C dryer.

### (3) Post-hybridization

After hybridization, the hybridization solution was lightly absorbed from the carbodiimidated cover glass , and post-hybridization washing was carried out under following condition.

The condition for post-hybridization washing was: (i) 2x SSC, at room temperature for 5 min; and (ii) 0.3 M aqueous ammonium citrate solution, at room temperature for 15 sec.

### (4) Detection of hybridization

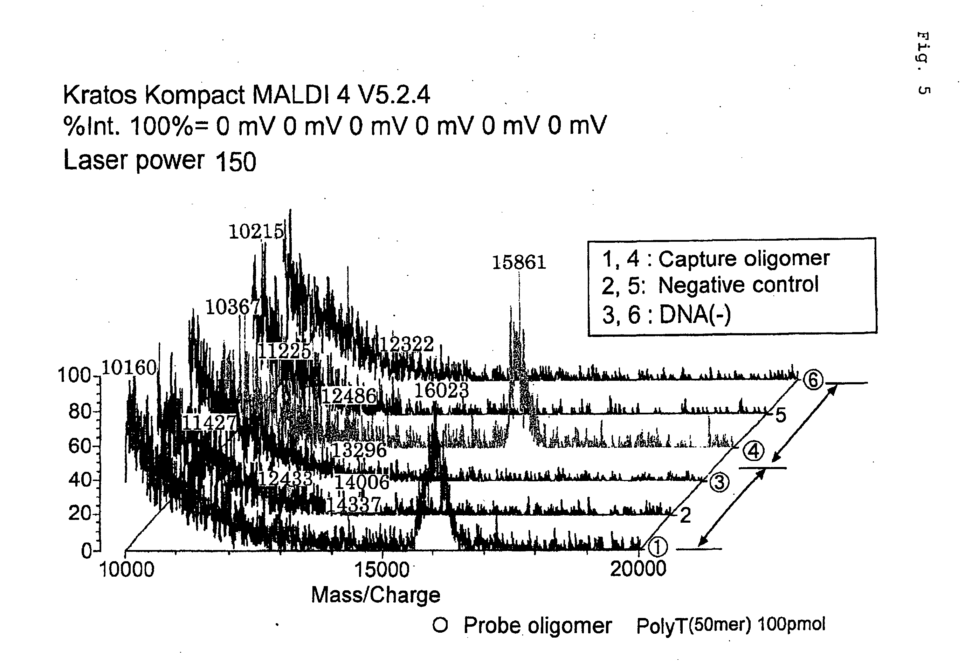

The obtained carbodiimidated glass sheet was measured using "KOMPACT MALDI 4 (Shimadzu Corp.)". The results of mass spectrometry are shown in Fig. 5. From samples containing complementary sequences, peaks were observed. Therefore, samples hybridized on a glass sheet were also found to be measurable by MALDI-TOF MS.

The matrix for the measurements was prepared by dissolving 0.0974 g of 0.7 M 3-hydroxypyridine-2-carboxylic acid (3-hydroxy-2-picolinic acid) (MW 139.11) and 0.010583 g of 0.07 M diammonium hydrogen citrate (MW 226.2) in 1 mL of 50% acetonitrile.

Furthermore, the measurement conditions for KOMPACT MALDI 4 are shown below.

| | |
|---|---|
| Flight path (ion flight path) | Linear |
| Polarity (ion polarity) | Negative |
| Mass (ion acceleration voltage) | 20 kV |
| Profiles (number of measurements per ion sample) | 50 |
| Aim (full range sample irradiation: 1-1000) | 1-1000 |
| Power (laser power) | 150 (50 mer), 180 (100 mer) |
| Accumulate | 10 |
| Average | 1 |
| Store profile | never |

### Industrial Applicability

The present invention provides a method for efficiently detecting genetic polymorphisms using a mass spectrometer. This method enables quick and comprehensive detection of genetic polymorphisms in a large number of specimens. The method of the present invention may greatly contribute towards disease correlation analysis and genetic diagnosis for determining polymorphisms that cause a specific disease.

## Claims

1. A method for detecting polymorphism in a DNA, which comprises the steps of:
(a) preparing from a subject, a DNA sample containing a polymorphic site and neighboring region thereof;
(b) hybridizing the above-mentioned DNA sample to an oligonucleotide fragment that can specifically hybridize to a DNA containing a target polymorphism, wherein said oligonucleotide fragment is immobilized on a platform; and
(c) detecting by mass spectrometry the DNA hybridized to the oligonucleotide fragment immobilized on the platform.

2. The method of claim 1, wherein the oligonucleotide fragment immobilized on the platform has a nucleotide sequence capable of specifically hybridizing to the nucleotide sequence of the neighboring region of the polymorphism to be detected.

3. The method of claim 1, wherein the DNA sample of step (a) can be obtained by: (i) performing polymerase chain reaction with a genomic DNA derived from a subject as a template using a primer pair containing a specifically cleavable site in at least one of the primers, which primer pair is designed to sandwich the polymorphic site between the primers, (ii) cleaving the obtained amplified product at the specifically cleavable site, and (iii) adding an arbitrary DNA to the amplified product after the cleavage, and wherein the oligonucleotide of step (b) is an oligonucleotide specifically hybridizing to the arbitrary DNA.

4. The method of claim 3, wherein the specifically cleavable site is a restriction enzyme site.

5. The method of any one of claims 1 to 4, wherein the polymorphism is a microsatellite.

6. The method of any one of claims 1 to 5, wherein the DNA sample containing the polymorphic site and neighboring region thereof is prepared using the polymorphic site of a plurality of different loci of the same person as the target, and the DNA sample is simultaneously hybridized to the oligonucleotide immobilized on the same platform.

7. The method of any one of claims 1 to 5, wherein the DNA sample containing the polymorphic site and neighboring region thereof is prepared using the polymorphic site of the same loci of a plurality of people as the target, and the DNA sample is simultaneously hybridized to the oligonucleotide immobilized on the same platform.

8. A platform to be used in the method of claim 3, which is immobilized with an oligonucleotide that specifically hybridizes to the arbitrary DNA.

9. A platform to be used in the method of claim 6, which is immobilized with an oligonucleotide fragment that hybridizes to the DNA sample containing the polymorphic site and neighboring region thereof, and which sample has been prepared using as the target the polymorphic site of a plurality of different loci of the same person.

10. A platform to be used in the method of claim 7, which is immobilized with an oligonucleotide fragment that hybridizes to the DNA sample containing the polymorphic site and neighboring region thereof, and which sample has been prepared using as the target the polymorphic site of the same loci of a plurality of people.

11. The platform of claim 9 or 10, wherein the oligonucleotide fragment has a sequence that specifically hybridizes to the sequence of the neighboring region of the polymorphism.

12. The platform of any one of claims 8 to 11, wherein the polymorphism is a microsatellite.

13. The platform of any one of claims 8 to 12, which is a glass sheet.
